# EUROPÄISCHE PATENTSCHRIFT

(11) **EP 2 373 562 B1**
(45) Veröffentlichungstag und Bekanntmachung des Hinweises auf die Patenterteilung: **03.10.2012**
(21) Anmeldenummer: 09774812.3
(22) Anmeldetag: 08.12.2009
(51) Int. Cl.: B65H 63/06

(54) **VERFAHREN UND VORRICHTUNG ZUR CHARAKTERISIERUNG EINES LÄNGLICHEN TEXTILEN PRÜFGUTS**
METHOD AND APPARATUS FOR CHARACTERIZING AN ELONGATED TEXTILE OBJECT TO BE TESTED
PROCÉDÉ ET DISPOSITIF DE CARACTÉRISATION D'UN ÉCHANTILLON TEXTILE ALLONGÉ

(30) Priorität: 07.01.2009 CH 16092009
(43) Veröffentlichungstag der Anmeldung: 12.10.2011
(73) Patentinhaber: Uster Technologies AG, 8610 Uster (CH)
(72) Erfinder: KELLER, Beat, CH-8046 Zürich (CH); GEHRIG, Stefan, CH-8610 Uster (CH); SCHMID, Peter, CH-8050 Zürich (CH); DE VRIES, Loris, 8616 Riedikon (CH)
(86) Internationale Anmeldenummer: PCT/CH2009/000390
(87) Internationale Veröffentlichungsnummer: WO 2010/078665

(56) Entgegenhaltungen:
- EP-A1- 0 685 580
- DE-A1- 10 348 741
- DE-A1-102007 028 651
- US-B1- 6 374 152

## Beschreibung

### FACHGEBIET

Die vorliegende Erfindung liegt auf dem Gebiet der textilen Qualitätskontrolle. Sie betrifft ein Verfahren und eine Vorrichtung zur Charakterisierung eines länglichen textilen Prüfguts, gemäss den Oberbegriffen der unabhängigen Patentansprüche. Derartige Verfahren und Vorrichtungen kommen typischerweise auf Spinn- oder Spulmaschinen zum Einsatz. Das längliche textile Prüfgut ist vorzugsweise Garn, kann aber auch ein Faserband oder ein Vorgarn etc. sein.

### STAND DER TECHNIK

Zur Sicherung der Garnqualität werden an Spinn- oder Spulmaschinen so genannte Garnreiniger eingesetzt. Eine derartige Vorrichtung ist z. B. aus der EP-1'249'422 A2 bekannt. Sie beinhaltet einen Messkopf mit mindestens einem Sensor, der das bewegte Garn abtastet. Häufig verwendete Sensorprinzipien sind das kapazitive (siehe z. B. EP-0'924'513 A1) oder das optische (siehe z. B. WO-93/13407 A1). Ziel der Abtastung ist es, Fehlstellen wie Dickstellen, Dünnstellen oder Fremdstoffe im Garn zu detektieren. Das Ausgangssignal des Sensors wird laufend mit vorgegebenen Bewertungskriterien bewertet. Die Bewertungskriterien werden üblicherweise in Form einer Reinigungsgrenze bzw. Reinigungskurve in einem zweidimensionalen Klassierfeld oder Ereignisfeld vorgegeben, welches von der Länge des Ereignisses einerseits und von einer Amplitude des Ereignisses, z. B. einer Abweichung der Garnmasse von einem Sollwert, andererseits aufgespannt wird. Ereignisse unterhalb der Reinigungsgrenze werden toleriert, Ereignisse oberhalb der Reinigungsgrenze werden aus dem Garn entfernt oder zumindest als Fehlstellen registriert.

Aus der US-6,374,152 B1 ist ein Verfahren zum Reinigen von Garnen bekannt, in dem die Reinigungsgrenze aufgrund der Dichte der Garnfehler selbsttätig berechnet und so optimal eingestellt wird. Zu diesem Zweck wird ein genügend langer Abschnitt des Garns vorgängig ausgemessen. Die gemessenen Ereignisse werden im bekannten zweidimensionalen Klassierfeld als Punkte oder Kreuze dargestellt. Aus den Messresultaten wird die Garnfehlerdichte berechnet und im Klassierfeld als Relief dargestellt. Die Reinigungsgrenze wird aufgrund einer vorgegebenen zulässigen Garnfehlerdichte selbsttätig festgelegt. Sie kann während des Kontrollvorgangs laufend nachgeregelt werden. Die Messungen erfolgen in den Garnreinigermessköpfen an den einzelnen Spinn- oder Spulstellen, während die Berechnungen und Regelungen in einer zentralen Recheneinheit stattfinden, mit der eine Vielzahl von Garnreinigermessköpfen verbunden ist.

Ein Nachteil des aus der US-6,374,152 B1 bekannten Verfahrens ist darin zu sehen, dass die grafischen Darstellungen aller Ereignisse und des Garnfehlerdichtereliefs im Klassierfeld wegen der vielen Informationen unübersichtlich sind. Ausserdem werden von den Garnreinigermessköpfen zur zentralen Recheneinheit grosse Datenmengen übermittelt. Man kann somit an die technischen Grenzen der Übertragungskapazität und der Datenverarbeitung stossen.

### DARSTELLUNG DER ERFINDUNG

Es ist eine Aufgabe der vorliegenden Erfindung, ein Verfahren und eine Vorrichtung zur Charakterisierung eines länglichen textilen Prüfguts wie Garn anzugeben, welche übersichtliche, relevante Daten über das textile Prüfgut liefern. Dadurch soll eine Bedienungsperson in die Lage versetzt werden, charakteristische Eigenschaften des textilen Prüfguts schnell zu erfassen und eine optimale Reinigungsgrenze rationell vorzugeben. Eine weitere Aufgabe ist es, die von den Garnreinigermessköpfen zur zentralen Recheneinheit zu übertragende Datenmenge zu reduzieren.

Ferner soll ein Verfahren zur Reinigung von länglichem textilem Prüfgut wie Garn angegeben werden, in welchem eine optimale Reinigungsgrenze schnell und rationell vorgegeben werden kann.

Diese und andere Aufgaben werden durch die erfindungsgemässen Verfahren und die erfindungsgemässe Vorrichtung gemäss den unabhängigen Patentansprüchen gelöst. Vorteilhafte Ausführungsformen sind in den abhängigen Patentansprüchen angegeben.

Nachfolgend wird die Erfindung ohne Einschränkung der Allgemeinheit am Beispiel von Garn erläutert. Sie lässt sich aber ebenso gut auf andere längliche textile Prüfgüter wie Faserband oder Vorgarn anwenden.

Erfindungsgemäss wird ein Abschnitt des Garns ausgemessen. Der Ausmessung entspringende Garndaten werden auf geeignete Weise aufbereitet, insbesondere zusammengefasst, vereinfacht und verdichtet. Eine grafische Darstellung der so aufbereiteten Garndaten verhilft der Bedienungsperson zu einem intuitiven Verständnis der Garnreinigung im Allgemeinen und der charakteristischen Eigenschaften des untersuchten Garns im Besonderen. Sie erlaubt somit der Bedienungsperson, charakteristische Eigenschaften des Garns schnell zu erfassen und die Reinigungsgrenze rationell vorzugeben. Dies wiederum führt zu einer in Bezug auf Qualität, Kosten und Zeit optimierten Garnreinigung.

Die Erfindung beruht auf der Erkenntnis, dass nicht alle im Garn gemessenen Ereignisse für die Beurteilung des Garns oder die Festlegung der Reinigungsgrenze gleich wichtig sind. Das Klassierfeld, das nachfolgend Ereignisfeld genannt wird, lässt sich diesbezüglich sehr grob in die folgenden drei Bereiche unterteilen:
(a) In der Nähe der beiden Achsen, d. h. für relativ kleine Ereignisamplituden und/oder relativ kleine Ereignislängen, treten viele Garnereignisse in hoher Dichte auf, die jedoch einzeln betrachtet eine kleine "Intensität" (z. B. Ereignislänge mal Ereignisamplitude) aufweisen und deshalb nicht stören. Diese Ereignisse können als statistisches "Rauschen" betrachtet werden. Sie brauchen nicht aus dem Garn entfernt zu werden. Dementsprechend sind sie auch für die Bedienungsperson nicht von Interesse und brauchen weder zur zentralen Recheneinheit übertragen noch im Ereignisfeld dargestellt zu werden. Dieser innere Bereich gehört gewissermassen zum "eigentlichen Garn" und wird deswegen als "Garnkörper" bezeichnet.
   Der Garnkörper charakterisiert das untersuchte Garn im folgenden Sinne:
   - Aus Grösse, Form und/oder Lage des Garnkörpers im Ereignisfeld lässt sich auf die Qualität des Garns und/oder das textile Erscheinungsbild einer daraus erzeugten textilen Fläche (Gewebe, Gestrick) schliessen.
   - Durch Entfernen von einzelnen oder in einem Schwarm auftretenden Ereignissen aus dem Garnkörper kann die Garnqualität nicht verbessert werden.
(b) An den Garnkörper grenzt ein mittlerer, bandförmiger Bereich an, in dem möglicherweise störende Garnfehler liegen. Die Ereignisdichte in diesem Bereich ist kleiner als im Garnkörper, aber immer noch beträchtlich. In diesen mittleren Bereich wird normalerweise die Reinigungsgrenze gelegt, welche die zulässigen von den unzulässigen Garnfehlern trennt. Hier sind also Lage und/oder Dichte der Ereignisse relevant.
(c) In grösserer Entfernung vom Garnkörper, d. h. für relativ grosse Ereignisamplituden und/oder relativ grosse Fehlerlängen, sind die Fehlerintensitäten zwar gross, aber ihre Dichte klein. Deshalb spielt dieser

- äussere Bereich für die Datenübermittlung und Datenverarbeitung eine untergeordnete Rolle.

Dementsprechend werden im erfindungsgemässen Verfahren zur Charakterisierung eines entlang seiner Längsrichtung bewegten länglichen textilen Prüfguts Messwerte einer Eigenschaft des textilen Prüfguts entlang der Längsrichtung des textilen Prüfguts erfasst. Aus den Messwerten werden Werte eines Parameters des textilen Prüfguts ermittelt. Es wird ein Ereignisfeld bereitgestellt, das einen Quadranten oder einen Teil eines Quadranten eines zweidimensionalen kartesischen Koordinatensystems beinhaltet, dessen Abszisse eine Erstreckung von Parameterwerten in der Längsrichtung und dessen Ordinate eine Abweichung des Parameters von einem Sollwert angibt. Aus den Werten des Parameters und ihrer Erstreckung in der Längsrichtung werden Dichten von Ereignissen in dem Ereignisfeld ermittelt. In dem Ereignisfeld wird ein Prüfgutkörper als Fläche dargestellt. Die Fläche wird einerseits durch die Abszisse oder eine parallel dazu verlaufende Gerade, andererseits durch die Ordinate oder eine parallel dazu verlaufende Gerade und ferner durch eine Linie in dem Ereignisfeld, welche im Wesentlichen einer konstanten Ereignisdichte folgt, begrenzt.

Die den Prüfgutkörper darstellende Fläche ist vorzugsweise zusammenhängend. Der Begriff "zusammenhängend" kann hier durchaus im Sinne der mathematischen Topologie verstanden werden. Dabei dürfte es in der Praxis keine Rolle spielen, ob der allgemeine Zusammenhang oder der speziellere Wegzusammenhang zur Definition verwendet wird. Die Fläche braucht aber nicht einfach zusammenhängend zu sein, d. h. sie darf allseitig umschlossene Aussparungen aufweisen.

Die gemessenen Parameter können sich auf die Masse pro Längeneinheit, auf den Durchmesser, auf die Reflektivität oder die Absorptivität des Prüfguts, auf Fremdstoffe im Prüfgut oder auf andere Eigenschaften des Prüfguts beziehen.

Die Grenzereignisdichte, welcher der den Prüfgutkörper begrenzenden Linie entspricht, sollte die folgenden Kriterien erfüllen:
- Sie ist so hoch, dass die Entfernung aller Ereignisse aus dem Prüfgut, die mit dieser oder einer tieferen Ereignisdichte vorkommen, die Produktivität zu stark beeinträchtigen würde, und
- sie ist gleichzeitig so tief, dass sie noch genügend nahe bei derjenigen Fehlerdichte liegt, die aus dem Prüfgut zu entfernen sinnvoll erscheint, z. B. zwischen zehn- und hundertmal höher.

Insbesondere für Garn geeignete Grenzereignisdichten liegen zwischen 500 und 2000 Ereignissen pro 100 km Prüfgutlänge und vorzugsweise bei 1000 Ereignissen pro 100 km Prüfgutlänge.

An die den Prüfgutkörper darstellende Fläche können sich eine oder mehrere weitere Flächen anschliessen, die ebenfalls von einer bzw. mehreren weiteren Linien in dem Ereignisfeld, welche jeweils im Wesentlichen einer konstanten Ereignisdichte folgen, begrenzt wird bzw. werden. Vorzugsweise bilden die Ereignisdichten, denen die den Prüfgutkörper begrenzende Linie und die weiteren Linien folgen, im Wesentlichen eine geometrische Folge.

Die den Prüfgutkörper darstellende Fläche und/oder die weiteren Flächen können sich grafisch von ihrer jeweiligen Umgebung unterscheiden, insbesondere indem sie eine andere Farbe, einen anderen Grauton und/oder ein anderes Muster aufweisen als ihre jeweilige Umgebung.

Die Werte des Garnparameters können mitsamt ihrer Erstreckung in der Längsrichtung als Ereignisse in dem Ereignisfeld dargestellt werden. Dabei werden vorzugsweise im Prüfgutkörper oder zumindest in einem an die Abszisse bzw. an die parallel dazu verlaufende Gerade angrenzenden Teil des Prüfgutkörpers keine Ereignisse dargestellt. Das Ereignisfeld kann hierzu durch eine Klassiergrenze in zwei Klassierbereiche aufgeteilt werden, wobei in einem ersten Klassierbereich keine Ereignisse dargestellt werden und in einem zweiten Klassierbereich Ereignisse dargestellt werden. Die Klassiergrenze folgt vorzugsweise im Wesentlichen einer konstanten Ereignisdichte.

Im erfindungsgemässen Verfahren zur Ausreinigung von Fehlern aus einem entlang seiner Längsrichtung bewegten länglichen textilen Prüfguts werden zur Charakterisierung des textilen Prüfguts Messwerte einer Eigenschaft des textilen Prüfguts entlang der Längsrichtung des textilen Prüfguts erfasst. Aus den Messwerten werden Werte eines Parameters des textilen Prüfguts ermittelt. Es wird ein Ereignisfeld bereitgestellt, das einen Quadranten oder einen Teil eines Quadranten eines zweidimensionalen kartesischen Koordinatensystems beinhaltet, dessen Abszisse eine Erstreckung von Parameterwerten in der Längsrichtung und dessen Ordinate eine Abweichung des Parameters von einem Sollwert angibt. Aus den Werten des Parameters und ihrer Erstreckung in der Längsrichtung werden Dichten von Ereignissen in dem Ereignisfeld ermittelt. In dem Ereignisfeld wird ein Prüfgutkörper als Fläche dargestellt, die einerseits durch die Abszisse oder eine parallel dazu verlaufende Gerade, andererseits durch die Ordinate oder eine parallel dazu verlaufende Gerade und ferner durch eine Linie in dem Ereignisfeld, welche im Wesentlichen einer konstanten Ereignisdichte folgt, begrenzt wird. Das Ereignisfeld wird durch mindestens eine Reinigungsgrenze bzw. Reinigungskurve in Reinigungsbereiche aufgeteilt, von denen ein erster Reinigungsbereich zulässige Ereignisse und ein zweiter Reinigungsbereich unzulässige Ereignisse vorgibt. Die zuvor vorgenommene Charakterisierung des textilen Prüfguts beeinflusst die Festlegung der Reinigungsgrenze.

Der erste Reinigungsbereich und der zweite Reinigungsbereich können sich grafisch voneinander unterscheiden, insbesondere indem sie unterschiedliche Farben, unterschiedliche Grautöne-und/oder unterschiedliche Muster aufweisen. Aufgrund der Messwerte des Garnparameters einerseits und der Reinigungsgrenze andererseits können die Anzahl unzulässiger Ereignisse und/oder die Anzahl unzulässiger Ereignisse pro Längeneinheit des textilen Prüfguts berechnet und ausgegeben werden. Dies sind wichtige Angaben für die Bedienungsperson, denn die Anzahl Schnitte pro Prüfgutlänge beeinflusst einerseits die Prüfgutqualität und andererseits die Produktivität. Die Anzahl _Schnitte pro Prüfgutlänge kann durch Ändern der Reinigungsgrenze vermindert oder erhöht werden. Durch probeweises Ändern der Reinigungsgrenze kann die Bedienungsperson diejenige Reinigungsgrenze finden, welche optimal den Bedürfnissen des Betriebs bezüglich Qualität, Kosten und Zeit entspricht. Dabei wird kein Prüfgut für Versuche verschwendet, denn die Versuche finden virtuell mittels Berechnungen auf der Grundlage der gemessenen Parameterwerte und ausgehend vom dargestellten Prüfgutkörper statt.

Im ersten Reinigungsbereich kann mindestens ein erster Ausnahmebereich definiert werden, der unzulässige Ereignisse vorgibt. Solche ersten Ausnahmebereiche können z. B. für Häufungen (Cluster) von Ereignissen definiert werden, die einzeln an sich zulässig wären, aber gehäuft stören würden und deshalb unerwünscht sind. Ebenso kann im zweiten Reinigungsbereich mindestens ein zweiter Ausnahmebereich definiert werden, der zulässige Ereignisse vorgibt. Solche zweiten Ausnahmebereiche können z. B. für Effekte definiert werden, die bei der Reinigung von Effektgarn nicht fälschlicherweise für Garnfehler gehalten werden und ausgereinigt werden dürfen.

Das Ereignisfeld braucht nicht auf einen einzigen Quadranten beschränkt zu sein. Es kann zusätzlich mindestens einen weiteren Quadranten oder einen Teil eines weiteren Quadranten beinhalten.

Zur numerischen Handhabung der Dichtekurven, welche für die Begrenzungen der den Prüfgutkörper darstellende Fläche und/oder die weiteren Flächen gebraucht werden, können bestimmte Stützwerte der jeweiligen Dichtekurve gespeichert werden. Zwischen den gespeicherten Stützwerten kann jeweils eine geeignete Interpolation, bzw. am Rand eine Extrapolation, durchgeführt werden.

Der Prüfgutkörper, die weiteren Flächen, die Klassierbereiche und/oder die Reinigungsbereiche können selbsttätig an die laufende Erfassung der Messwerte und an die sich daraus ergebenden Änderungen der Dichten von Ereignissen in dem Ereignisfeld angepasst werden.

Die erfindungsgemässe Vorrichtung zur Charakterisierung eines entlang seiner Längsrichtung bewegten länglichen textilen Prüfguts beinhaltet einen Messkopf zur Erfassung von Messwerten einer Eigenschaft des textilen Prüfguts entlang der Längsrichtung des textilen Prüfguts sowie zur Ermittlung von Werten eines Parameters des textilen Prüfguts aus den Messwerten. Sie beinhaltet ferner eine mit dem Messkopf verbundene Steuereinheit. Die Steuereinheit weist eine Speichereinheit und eine Ausgabeeinheit zur Speicherung bzw. Ausgabe eines Ereignisfeldes, das einen Quadranten oder einen Teil eines Quadranten eines zweidimensionalen kartesischen Koordinatensystems beinhaltet, dessen Abszisse eine Erstreckung von Parameterwerten in der Längsrichtung und dessen Ordinate eine Abweichung des Parameters von einem Sollwert angibt, und eine Recheneinheit zur Ermittlung von Dichten von Ereignissen in dem Ereignisfeld aus den Werten des Parameters und ihrer Erstreckung in der Längsrichtung auf. Die Steuereinheit ist zur Ausgabe eines Prüfgutkörpers als Fläche in dem Ereignisfeld eingerichtet. Die Fläche wird einerseits durch die Abszisse oder eine parallel dazu verlaufende Gerade, andererseits durch die Ordinate oder eine parallel dazu verlaufende Gerade und ferner durch eine Linie in dem Ereignisfeld, welche im Wesentlichen einer konstanten Ereignisdichte folgt, begrenzt.

Die erfindungsgemässe Vorrichtung kommt in einer Textilverarbeitungsmaschine, bspw. einer Spinn- oder Spulmaschine für Garn, zum Einsatz. Eine derartige Textilverarbeitungsmaschine weist typischerweise eine Vielzahl von Arbeitsstellen auf. Dementsprechend kann die erfindungsgemässe Vorrichtung eine Vielzahl von Messköpfen beinhalten, die sich an jeder Arbeitsstelle befinden. Die Messköpfe sind alle an der zentralen Steuereinheit angeschlossen, z. B. über einen seriellen Bus wie z. B. RS-485. Zwischen einem jeweiligen Messkopf und der Steuereinheit kann ein Schnittstellenwandler eingebaut sein. Die Steuereinheit ist vorzugsweise in der Textilverarbeitungsmaschine eingebaut. Dies hat den Vorteil, dass die Person, welche die Textilverarbeitungsmaschine bedient, die Einstellung der Reinigungsgrenze an ihrem Arbeitsplatz vornehmen kann, und dass kein zusätzlicher Rechner benötigt wird. Die erfindungsgemässe Vorrichtung kann an jeder Arbeitsstelle, in der Nähe eines jeden Messkopfes, eine Schneideinrichtung zur Entfernung von unzulässigen Ereignissen aus dem Garn beinhalten.

### AUFZÄHLUNG DER ZEICHNUNGEN

Nachfolgend wird die Erfindung am Beispiel einer Spulmaschine für Garn anhand von Zeichnungen detailliert erläutert.
- Figur 1: zeigt schematisch eine Spulmaschine mit einem Garnreinigersystem.
- Figur 2: zeigt ein Ereignisfeld mit einem Garnkörper und einer Reinigungskurve, wie es sich aus dem erfindungsgemässen Verfahren ergeben kann.
- Figur 3: zeigt ein Ereignisfeld mit Garnereignissen, einem Garnkörper und einer Reinigungsgrenze, wie sie sich aus dem erfindungsgemässen Verfahren ergeben können.
- Figur 4: zeigt ein weiteres Ereignisfeld mit einem Garnkörper und einer Reinigungskurve, wie es sich aus dem erfindungsgemässen Verfahren ergeben kann.

### AUSFÜHRUNG DER ERFINDUNG

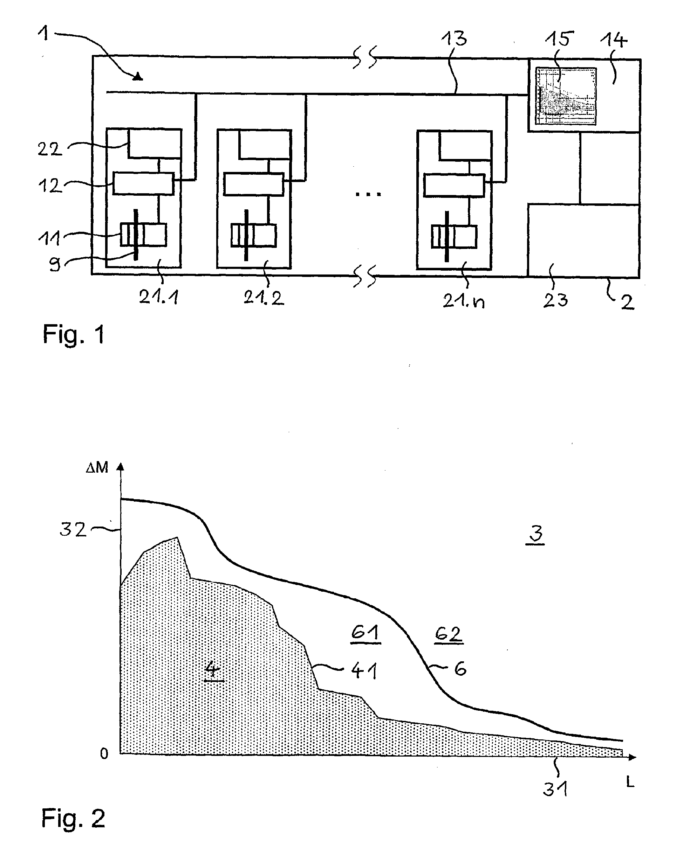

In **Figur 1** ist sehr schematisch eine Spulmaschine 2 mit mehreren Spulstellen 21.1, 21.2, ..., 21.n dargestellt. Eine erfindungsgemässe Vorrichtung 1 ist in die Spulmaschine 2 eingebaut. An jeder Spulstelle 21.1 wird während des Umspulvorgangs Garn 9 von einem Messkopf 11 der erfindungsgemässen Vorrichtung 1 überwacht. Der Messkopf 11 beinhaltet einen Sensor, mit dem eine Eigenschaft des Garns 9 gemessen wird, z. B. einen kapazitiven Sensor zur Messung einer dielektrischen Eigenschaft des Garns 9. Ferner beinhaltet der Messkopf 11 eine Auswerteeinheit, die dazu eingerichtet ist, aus den Messwerten einen Garnparameter, z. B. die Garnmasse pro Längeneinheit, zu ermitteln. Der Messkopf 11 ist über einen Schnittstellenwandler 12 mit einer zentralen Steuereinheit 14 der erfindungsgemässen Vorrichtung 1 verbunden. Über die Verbindung wird der Messkopf 11 von der Steuereinheit 14 eingestellt und gesteuert, und der Messkopf 11 übermittelt Daten wie die ermittelten Garnparameter an die Steuereinheit 14. Eine Verbindungsleitung 13 zwischen allen Schnittstellenwandlern 12 und der Steuereinheit 14 kann als serieller Bus wie z. B. RS-485 ausgebildet sein. Der Schnittstellenwandler 12 kann zusätzlich auch mit einem Spulstellenrechner 22 der jeweiligen Spulstelle 21.1 verbunden sein. Die Steuereinheit 14 weist eine Ausgabeeinheit und eine Eingabeeinheit für eine Bedienungsperson auf. Vorzugsweise sind Ausgabe- und Eingabeeinheit gemeinsam als Sensorbildschirm (Touchscreen) 15 ausgebildet. Die Steuereinheit 14 ist mit einem Steuerrechner 23 der Spulmaschine 2 verbunden. Statt in der Steuereinheit 14 können die Ausgabe- und/oder die Eingabeeinheit in der Spulmaschine 2, z. B. im Steuerrechner 23, eingebaut sein.

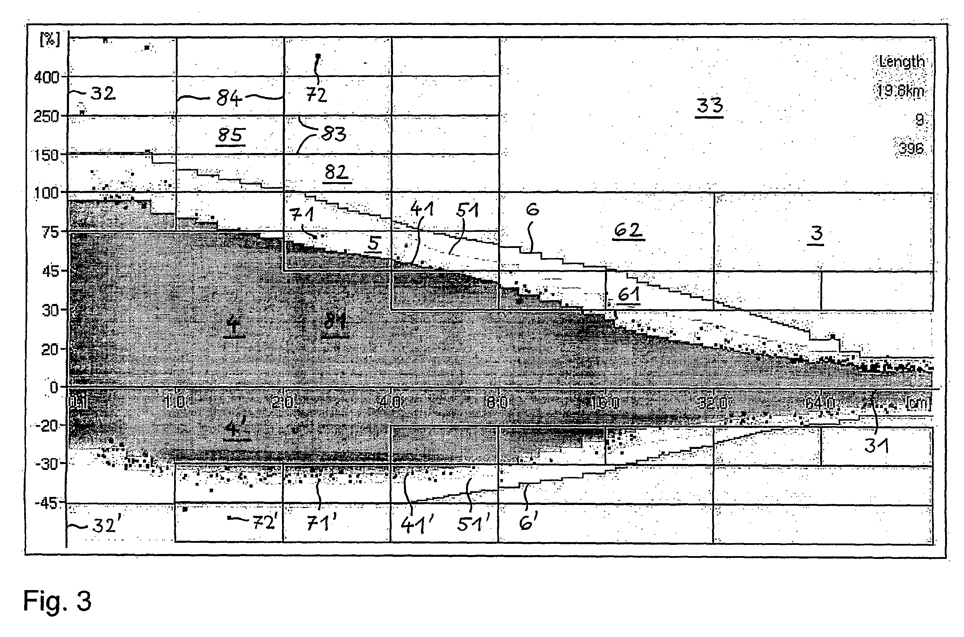

**Figur 2** zeigt ein mögliches Ereignisfeld 3 mit einem Garnkörper und einer Reinigungsgrenze 6, wie es auf der Ausgabeeinheit 15 der Steuereinheit 14 dargestellt werden kann. Das Ereignisfeld 3 ist ein Quadrant eines zweidimensionalen kartesischen Koordinatensystems, das durch eine Abszisse 31 und eine Ordinate 32 aufgespannt wird. Die Ordinate 32 gibt eine Abweichung ΔM des Garnparameters, z. B. die Abweichung der Garnmasse pro Längeneinheit, von einem Sollwert an. Der Sollwert wird vorzugsweise durch eine laufende Mittelwertbildung über eine Vielzahl von Messungen ermittelt. Die Abszisse 31 gibt an, über welche Länge L entlang der Garnlängsrichtung sich eine Abweichung ΔM erstreckt. Eine ermittelte Abweichung ΔM und ihre Länge L bilden zusammen die Koordinaten eines Gamereignisses, das einen Punkt im Ereignisfeld 3 definiert und dort auf geeignete Weise dargestellt werden kann (vgl. Fig. 3).

In einem Einmessvorgang wird ein genügend langer Garnabschnitt ausgemessen. Als "genügend" wird eine Einmesslänge von mindestens ca. 1 km angesehen; grössere Einmesslängen von z. B. 10 km oder 100 km Länge werden aber bevorzugt, weil sie statistisch aussagekräftigere Resultate liefern. Die Werte des Garnparameters ΔM und die dazugehörigen Längen L werden vom Messkopf 11 an die Steuereinheit 14 übermittelt. In einer Recheneinheit der Steuereinheit 14 werden daraus Dichten von Ereignissen im Ereignisfeld 3 ermittelt, so wie es z. B. in der US-6,374,152 B1 beschrieben ist. Auf diese Weise kann jedem Punkt des Ereignisfeldes 3 eindeutig eine Ereignisdichte zugeordnet werden. Durch Interpolation, Extrapolation, Glättung und/oder andere numerische Verfahren können zu abrupte lokale Änderungen der so ermittelten Ereignisdichtefunktion, die möglicherweise durch Messfehler oder sonstige Artefakte verursacht sind, vermieden werden.

Erfindungsgemäss wird aus der Ereignisdichtefunktion ein Garnkörper berechnet und als Fläche 4 im Ereignisfeld 3 dargestellt. Es wird eine genügend hohe Grenzereignisdichte von z. B. 1000 Ereignissen pro 100 km Garnlänge gewählt. Die Verbindung aller Punkte im Ereignisfeld 3, denen die Grenzereignisdichte zugeordnet ist, ergibt eine Dichtekurve 41, welche den Garnkörper vom übrigen Ereignisfeld 3 abgrenzt. Gegen die beiden Koordinatenachsen 31, 32 hin wird der Garnkörper durch die Koordinatenachsen 31, 32 selbst begrenzt. Durch diese Abgrenzungen entsteht eine zusammenhängende Fläche 4, die für das ausgemessene Garn 9 charakteristisch ist. Die den Garnkörper darstellende Fläche 4 unterscheidet sich grafisch vom übrigen Ereignisfeld 3, indem sie z. B. eine andere Farbe, einen anderen Grauton und/oder ein anderes Muster aufweist als das übrige Ereignisfeld 3.

Die erfindungsgemässe Berechnung und Darstellung des Garnkörpers erlauben es der Bedienungsperson, die Charakteristika des untersuchten Garns 9 schnell und intuitiv zu erfassen. Dies ermöglicht es der Bedienungsperson, schnell und rationell eine Reinigungskurve 6 für die Ausreinigung von Fehlern aus dem Garn 9 festzulegen. Die Reinigungskurve 6 ist diejenige Linie, welche das Ereignisfeld 3 in Reinigungsbereiche 61, 62 aufteilt, von denen ein erster Reinigungsbereich 61 zulässige Ereignisse und ein zweiter Reinigungsbereich 62 unzulässige Ereignisse vorgibt. Sie ist die grafische Darstellung einer Reinigungsgrenze, d. h. eines Kriteriums für die Beurteilung der Garnqualität. Die Festlegung der Reinigungsgrenze bzw. Reinigungskurve 6 erfolgt vorzugsweise über die Eingabeeinheit 15 an der Steuereinheit 14. Dabei kann die Steuereinheit 14 eine Standard-Reinigungskurve vorschlagen, etwa als eine weitere Dichtekurve mit deutlich tieferer Ereignisdichte als die Grenzereignisdichte 41 für den Garnkörper 4. Die Reinigungskurve 6 sollte über dem Garnkörper 4 liegen, weil es unsinnig wäre, Ereignisse aus dem Garnkörper 4 ausreinigen zu wollen. Die Bedienungsperson kann die Standard-Reinigungskurve durch Eingaben an der Eingabeeinheit 15 ändern. Alternativ kann die Bedienungsperson durch Eingaben an der Eingabeeinheit 15 die Reinigungskurve 6 nach eigenen Erfahrungen definieren, ohne von einem Vorschlag der Steuereinheit 14 auszugehen.

Figur 3 zeigt eine erweiterte Darstellung eines Ereignisfeldes 3, wie sie auf der Ausgabeeinheit 15 der Steuereinheit 14 ausgegeben werden kann. Hier beinhaltet das Ereignisfeld 3 nicht nur den ersten, sondern zusätzlich auch noch den vierten Quadranten. Im ersten Quadranten werden durch lokale Massenzunahme verursachte Dickstellen, im vierten Quadranten durch lokale Massenabnahme verursachte Dünnstellen gegenüber der jeweiligen Länge L aufgetragen. Dadurch, dass die Längenachsen 31 der beiden Quadranten zusammenfallen, fügen sich der Garnkörper 4 für die Dickstellen und der Garnkörper 4' für die Dünnstellen visuell zu einem einzigen Gesamt-Garnkörper zusammen. Dieser ist bezüglich der Abszisse 31 nicht notwendigerweise symmetrisch. Die Achsen 31, 32, 32' für Länge und Massenzunahme bzw. Massenabnahme sind vorzugsweise logarithmisch, fast logarithmisch oder teilweise logarithmisch eingeteilt.

Im Beispiel von Figur 3 schliesst sich an die den Garnkörper darstellende Fläche 4 eine weitere Fläche 5 an. Die weitere Fläche 5 wird durch die den Garnkörper begrenzende Dichtekurve 41 und durch eine weitere Dichtekurve 51, die einer tieferen Ereignisdichte entspricht, begrenzt. Die höhere Ereignisdichte kann z. B. 1000 Ereignisse pro 100 km Garnlänge und die tiefere Ereignisdichte z. B. 100 Ereignisse pro 100 km Garnlänge betragen. Diese beiden Ereignisdichten sind somit Teil der geometrischen Folge 1, 10, 100, 1000, ... Ereignisse pro 100 km Garnlänge. Die den Garnkörper darstellende Fläche 4 und die weitere Fläche 5 unterscheiden sich grafisch voneinander und von ihrer jeweiligen Umgebung. So können z. B. die den Garnkörper darstellende Fläche 4 dunkelgrün und die weitere Fläche 5 hellgrün eingefärbt sein. Statt nur einer weiteren Fläche 5 könnten auch mehrere weitere Flächen dargestellt werden. Solche weiteren Flächen 5 können der Bedienungsperson helfen, den Charakter des Garns 9 noch besser zu erfassen und die Reinigungsgrenze 6 optimal vorzugeben.

Im Ereignisfeld 3 von Figur 3 ist auch eine Reinigungskurve 6 eingezeichnet. Sie trennt einen ersten Reinigungsbereich 61, der zulässige Ereignisse vorgibt, und einen zweiten Reinigungsbereich 62, der unzulässige Ereignisse vorgibt, voneinander. Die beiden Reinigungsbereiche 61, 62 sind vorzugsweise unterschiedlich eingefärbt, z. B. der erste Reinigungsbereich 61 weiss und der zweite Reinigungsbereich 62 grau. Die Reinigungskurve 6 ist grundsätzlich von den Dichtekurven 41, 51 unabhängig, obwohl sie sich in ihrem groben Verlauf an sie halten wird. In Figur 3 fällt die Reinigungskurve 6 teilweise mit der weiteren Dichtekurve 51 zusammen, teilweise nicht.

Ferner sind im Ereignisfeld 3 von Figur 3 Ereignisse 71, 72 in Form von Quadraten eingezeichnet. Dabei können sich die Symbole für die zulässigen Ereignisse 71 im ersten Reinigungsbereich 61 diesseits der Reinigungskurve 6 von denjenigen für die unzulässigen Ereignisse 72 im zweiten Reinigungsbereich 62 jenseits der Reinigungskurve 6 unterscheiden, z. B. durch ihre Grösse und/oder ihre Farbe. Die Ereignisse bilden zusammen eine so genannte Punktewolke (englisch scatter plot).

Nicht alle gemessenen Ereignisse werden im Ereignisfeld 3 eingetragen. Der grösste Teil des Garnkörpers ist frei von Ereignissen. Grund dafür ist, dass Ereignisse im Garnkörper für eine Bedienungsperson schlicht uninteressant sind. Sie "gehören zum Garn". Das Nichtdarstellen derjenigen Ereignisse, die in der Nähe der Abszisse 31 liegen, hat zumindest die folgenden beiden Vorteile:
- Diese Ereignisse brauchen nicht vom Messkopf 11 zur Steuereinheit 14 übertragen zu werden. Somit wird die zu übertragende und zu verarbeitende Datenmenge wesentlich reduziert und die Datenleitungen sowie die Steuereinheit 14 entlastet. Die dadurch frei gewordenen Kapazitäten können für Wichtigeres genutzt werden.
- Die grafische Darstellung enthält weniger Ereignispunkte und wird dadurch übersichtlicher. Die Bedienungsperson wird nicht von unnötigen Ereignispunkten abgelenkt und kann sich auf das Wesentliche konzentrieren.

Das Ereignisfeld 3 ist also in zwei Klassierbereiche 81, 82 aufgeteilt: in einen ersten Klassierbereich 81, in dem keine Ereignisse dargestellt werden, und in einen zweiten Klassierbereich 82, in dem Ereignisse dargestellt werden. Die beiden Klassierbereiche 81, 82 sind durch eine Klassiergrenze voneinander abgegrenzt. Diese Klassiergrenze ist im Beispiel von Figur 3 nicht eingezeichnet. Ihr Verlauf lässt sich nur anhand der noch dargestellten Ereignisse 71 erahnen. Sie folgt vorzugsweise auch einer Dichtekurve, die einer höheren, gleichen oder niedrigeren Ereignisdichte entsprechen kann als die den Garnkörper 4 begrenzende Dichtekurve 41.

Der zweite Klassierbereich 82 ist durch waagrechte Klassengrenzen 83 und senkrechte Klassengrenzen 84 in rechteckige Klassen 85 für Garnereignisse 71, 72 unterteilt. Solche

Klassen 85 dienen in erster Linie der besseren statistischen Erfassung ähnlicher Ereignisse 71, 72. So können z. B. die festgestellten Ereignisse 71, 72 in jeder Klasse 85gezählt und die Resultate klassenweise ausgegeben werden. Sinnvollerweise werden die Klassen 85 nur im zweiten Klassierbereich 82, in dem Ereignisse überhaupt dargestellt werden, angezeigt. Es wäre aber möglich, das ganze Ereignisfeld 3 in solche Klassen 85 zu unterteilen.

Was oben für den ersten Quadranten des Ereignisfeldes 3 von Figur 3 beschrieben wurde, gilt analog auch für den vierten Quadranten. Die entsprechenden Elemente im vierten Quadranten sind mit demselben Bezugszeichen wie im ersten Quadranten und einem zusätzlichen Apostroph bezeichnet.

In einem Beschreibungsfeld 33 findet man in Figur 3 die folgenden beispielhaften Angaben:
- Die Garnlänge, aufgrund derer die Dichtekurven 41, 42 für den Garnkörper 4 und die weitere Fläche 5 ermittelt wurden, betrug 19.8 km.
- Die Anzahl Ereignisse 72, die in den beiden zweiten Reinigungsbereichen 62 liegen und somit als Fehler aus dem Garnabschnitt von 19.8 km Länge entfernt würden, beträgt 9. Somit ergäben sich 45 Schnitte pro 100 km Garnlänge, welche Schnittzahl bei Bedarf ebenfalls angezeigt werden könnte.
- Die Gesamtzahl im Ereignisfeld 3 eingetragener Ereignisse 71, 72 beträgt 396.

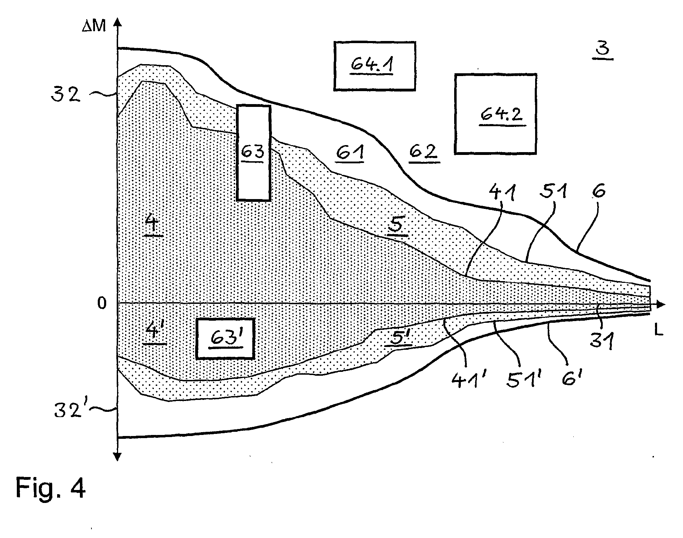

Eine weitere Darstellung eines Ereignisfeldes 3 zeigt Figur 4. Viele Elemente sind schon aus den Figuren 2 und/oder 3 bekannt, werden in Figur 4 mit denselben Bezugszeichen bezeichnet und brauchen hier nicht erneut erläutert zu werden, so etwa der vierte Quadrant für Dünnstellen, der Garnkörper 4, die weitere Fläche 5 und die Reinigungskurve 6. Im Unterschied zu den vorhergehenden Figuren 2 und 3 sind im Ereignisfeld 3 der Figur 4 Ausnahmebereiche 63, 63', 64.1, 64.2 definiert. In einem solchen Ausnahmebereich 63, 63', 64.1, 64.2 wird das Zulässigkeitskriterium für Garnereignisse 71, 72 gegenüber seiner Umgebung invertiert, d. h.:
- Ereignisse, die in einem ersten, im ersten Reinigungsbereich 61 liegenden Ausnahmebereich 63, 63' liegen, sind unzulässig. Solche ersten Ausnahmebereiche 63, 63' können z. B. für Häufungen (Cluster) von Ereignissen 71 definiert werden, die einzeln an sich zulässig wären, aber gehäuft stören würden und deshalb unerwünscht sind. Die ersten Ausnahmebereiche 63, 63' können sich vollständig oder teilweise im Garnkörper 4 oder ausserhalb des Garnkörpers 4 befinden, jedenfalls aber im ersten Reinigungsbereich 61. Der Garnkörper 4', der sich im vierten Quadranten befindet, bildet mit seinem allseitig umschlossenen ersten Ausnahmebereich 63' ein Beispiel für eine 2-zusammenhängende Fläche im Sinne der mathematischen Topologie. Dagegen sind die vorher diskutierten Garnkörper 4 einfach zusammenhängend und auch wegzusammenhängend.
- Ereignisse, die in einem zweiten, im zweiten Reinigungsbereich 62 liegenden Ausnahmebereich 64.1, 64.2 liegen, sind zulässig. Solche zweiten Ausnahmebereiche 64.1, 64.2 können z. B. für Effekte definiert werden, die bei der Reinigung von Effektgarn nicht fälschlicherweise für Garnfehler gehalten werden und ausgereinigt werden dürfen.

### BEZUGSZEICHENLISTE

- 1: Vorrichtung
- 11: Messkopf
- 12: Schnittstellenwandler
- 13: Verbindungsleitung
- 14: Steuereinheit
- 15: Sensorbildschirm als Eingabe- und Ausgabeeinheit

- 2: Spulmaschine
- 21.1, 21.2, ...: Spulstellen
- 22: Spulstellenrechner
- 23: Steuerrechner

- 3: Ereignisfeld
- 31: Abszisse
- 32: Ordinate

- 4: Garnkörper, die den Garnkörper darstellende Fläche
- 41: das Ereignisfeld abgrenzende Dichtekurve

- 5: weitere Fläche
- 51: die weitere Fläche abgrenzende Dichtekurve

- 6: Reinigungsgrenze bzw. Reinigungskurve
- 61: erster Reinigungsbereich
- 62: zweiter Reinigungsbereich
- 63: erster Ausnahmebereich
- 64.1,64.2: zweiter Ausnahmebereich

- 71: zulässiges Ereignis
- 72: unzulässiges Ereignis

- 81: erster Klassierbereich
- 82: zweiter Klassierbereich
- 83: waagrechte Klassengrenze
- 84: senkrechte Klassengrenze
- 85: Klasse

- 9: Garn

## Patentansprüche

1. Verfahren zur Charakterisierung eines entlang seiner Längsrichtung bewegten länglichen textilen Prüfguts (9), wobei
Messwerte einer Eigenschaft des textilen Prüfguts (9) entlang der Längsrichtung des textilen Prüfguts (9) erfasst werden,
Werte eines Parameters des textilen Prüfguts aus den Messwerten ermittelt werden, ein Ereignisfeld (3) bereitgestellt wird, das einen Quadranten oder einen Teil eines Quadranten eines zweidimensionalen kartesischen Koordinatensystems beinhaltet, dessen Abszisse (31) eine Erstreckung (L) von Parameterwerten in der Längsrichtung und dessen Ordinate (32) eine Abweichung (ΔM) des Parameters von einem Sollwert angibt, und
aus den Werten des Parameters und ihrer Erstreckung (L) in der Längsrichtung Dichten von Ereignissen (71, 72) in dem Ereignisfeld (3) ermittelt werden,
**dadurch gekennzeichnet,**
**dass** in dem Ereignisfeld (3) ein Prüfgutkörper als Fläche (4) dargestellt wird, die
einerseits durch die Abszisse (31) oder eine parallel dazu verlaufende Gerade, andererseits durch die Ordinate (32) oder eine parallel dazu verlaufende Gerade und
ferner durch eine Linie (41) in dem Ereignisfeld (4), welche im Wesentlichen einer konstanten Ereignisdichte folgt,
begrenzt wird.

2. Verfahren nach Anspruch 1, wobei die konstante Ereignisdichte zwischen 500 und 2000 Ereignissen pro 100 km Prüfgutlänge und vorzugsweise bei 1000 Ereignissen pro 100 km Prüfgutlänge liegt.

3. Verfahren nach einem der vorangehenden Ansprüche, wobei sich an die den Prüfgutkörper darstellende Fläche (4) eine oder mehrere weitere Flächen (5) anschliessen, die ebenfalls von einer bzw. mehreren weiteren Linien (51) in dem Ereignisfeld (3), welche jeweils im Wesentlichen einer konstanten Ereignisdichte folgen, begrenzt wird bzw. werden.

4. Verfahren nach Anspruch 3, wobei die Ereignisdichten, denen die den Prüfgutkörper (4) begrenzende Linie (41) und die weiteren Linien (51) folgen, im Wesentlichen eine geometrische Folge bilden.

5. Verfahren nach einem der vorangehenden Ansprüche, wobei sich die den Prüfgutkörper darstellende Fläche (4) und/oder die weiteren Flächen (5) grafisch von ihrer jeweiligen Umgebung unterscheiden, insbesondere indem sie eine andere Farbe, einen anderen Grauton und/oder ein anderes Muster aufweisen als ihre jeweilige Umgebung.

6. Verfahren nach einem der vorangehenden Ansprüche, wobei die Werte des Parameters mitsamt ihrer Erstreckung (L) in der Längsrichtung als Ereignisse (71, 72) in dem Ereignisfeld (3) dargestellt werden.

7. Verfahren nach Anspruch 6, wobei im Prüfgutkörper (4) oder zumindest in einem an die Abszisse (31) bzw. an die parallel dazu verlaufende Gerade angrenzenden Teil des Prüfgutkörpers (4) keine Ereignisse dargestellt werden.

8. Verfahren nach Anspruch 7, wobei das Ereignisfeld (3) durch eine Klassiergrenze in zwei Klassierbereiche (81, 82) aufgeteilt wird, und in einem ersten Klassierbereich (81) keine Ereignisse dargestellt werden und in einem zweiten Klassierbereich (82) Ereignisse (71, 72) dargestellt werden.

9. Verfahren nach Anspruch 8, wobei die Klassiergrenze im Wesentlichen einer konstanten Ereignisdichte folgt.

10. Verfahren zur Ausreinigung von Fehlern (72) aus einem entlang seiner Längsrichtung bewegten länglichen textilen Prüfgut (9), wobei Messwerte einer Eigenschaft des textilen Prüfguts (9) entlang der Längsrichtung des textilen Prüfguts (9) erfasst werden,
Werte eines Parameters des textilen Prüfguts aus den Messwerten ermittelt werden, ein Ereignisfeld (3) bereitgestellt wird,
das einen Quadranten oder einen Teil eines Quadranten eines zweidimensionalen kartesischen Koordinatensystems beinhaltet, dessen Abszisse (31) eine Erstreckung (L) von Parameterwerten in der Längsrichtung und dessen Ordinate (32) eine Abweichung (ΔM) des Parameters von einem Sollwert angibt, und
das durch mindestens eine Reinigungskurve (6) in Reinigungsbereiche (61, 62) aufgeteilt wird, von denen ein erster Reinigungsbereich (61) zulässige Ereignisse (71) und ein zweiter Reinigungsbereich (62) unzulässige Ereignisse (72) vorgibt, und
aus den Werten des Parameters und ihrer Erstreckung (L) in der Längsrichtung Dichten von Ereignissen in dem Ereignisfeld (3) ermittelt werden,
**dadurch gekennzeichnet,**
**dass** das textile Prüfgut (9) nach einem der vorangehenden Ansprüche charakterisiert wird und
die Charakterisierung die Festlegung der Reinigungskurve (6) beeinflusst.

11. Verfahren nach Anspruch 10, wobei sich der erste Reinigungsbereich (61) und der zweite Reinigungsbereich (62) grafisch voneinander unterscheiden, insbesondere indem sie unterschiedliche Farben, unterschiedliche Grautöne und/oder unterschiedliche Muster aufweisen.

12. Verfahren nach Anspruch 10 oder 11, wobei aufgrund der Messwerte des Garnparameters und aufgrund der Reinigungskurve (6) die Anzahl unzulässiger Ereignisse (72) und/oder die Anzahl unzulässiger Ereignisse (72) pro Längeneinheit des textilen Prüfguts (9) berechnet und ausgegeben werden.

13. Verfahren nach einem der Ansprüche 10-12, wobei im ersten Reinigungsbereich (61) mindestens ein erster Ausnahmebereich (63) definiert wird, der unzulässige Ereignisse vorgibt, und/oder im zweiten Reinigungsbereich (62) mindestens ein zweiter Ausnahmebereich (64.1, 64.2) definiert wird, der zulässige Ereignisse vorgibt.

14. Verfahren nach einem der vorangehenden Ansprüche, wobei das Ereignisfeld (3) zusätzlich mindestens einen weiteren Quadranten oder einen Teil eines weiteren Quadranten beinhaltet.

15. Verfahren nach einem der vorangehenden Ansprüche, wobei der Prüfgutkörper (4), die weiteren Flächen (5), die Klassierbereiche (81, 82) und/oder die Reinigungsbereiche (61, 62) selbsttätig an die laufende Erfassung der Messwerte und an die sich daraus ergebenden Änderungen der Dichten von Ereignissen (71, 72) in dem Ereignisfeld (3) angepasst werden.

16. Vorrichtung (1) zur Charakterisierung eines entlang seiner Längsrichtung bewegten länglichen textilen Prüfguts (9), beinhaltend
einen Messkopf (11) zur Erfassung von Messwerten einer Eigenschaft des textilen Prüfguts (9) entlang der Längsrichtung des textilen Prüfguts (9) sowie zur Ermittlung von Werten eines Parameters des textilen Prüfguts aus den Messwerten, und
eine mit dem Messkopf (11) verbundene Steuereinheit (14)
mit einer Speichereinheit und einer Ausgabeeinheit (15) zur Speicherung bzw. Ausgabe eines Ereignisfeldes (3), das einen Quadranten oder einen Teil eines Quadranten eines zweidimensionalen kartesischen Koordinatensystems beinhaltet, dessen Abszisse (31) eine Erstreckung (L) von Parameterwerten in der Längsrichtung und dessen Ordinate (32) eine Abweichung (ΔM) des Parameters von einem Sollwert angibt, und
einer Recheneinheit zur Ermittlung von Dichten von Ereignissen (71, 72) in dem Ereignisfeld (3) aus den Werten des Parameters und ihrer Erstreckung (L) in der Längsrichtung,
**dadurch gekennzeichnet,**
**dass** die Steuereinheit (14) zur Ausgabe eines Prüfgutkörpers als Fläche (4) in dem Ereignisfeld (3) eingerichtet ist, die
einerseits durch die Abszisse (31) oder eine parallel dazu verlaufende Gerade, andererseits durch die Ordinate (32) oder eine parallel dazu verlaufende Gerade und
ferner durch eine Linie (41) in dem Ereignisfeld (3), welche im Wesentlichen einer konstanten Ereignisdichte folgt,
begrenzt wird.

## Claims

1. A method for characterizing an elongate textile product to be tested (9) which is moved along its longitudinal direction, wherein
readings of a characteristic of the textile product to be tested (9) are detected along the longitudinal direction of the textile product to be tested (9),
values of a parameter of the textile product to be tested are determined from the readings,
an event field (3) is provided, which contains a quadrant or a part of a quadrant of a two-dimensional Cartesian coordinate system, whose abscissa (31) defines an extension (L) of parameter values in the longitudinal direction and whose ordinate (32) defines a deviation (ΔM) of the parameter from a desired value, and
densities of events (71, 72) in the event field (3) are determined from the values of the parameter and their extensions (L) in the longitudinal direction,
**characterised in that**
a body of the product to be tested is represented in the event field (3) as an area (4) which is delimited
on the one hand by the abscissa (31) or a straight line running parallel thereto,
on the other hand by the ordinate (32) or a straight line running parallel thereto, and further by a line (41) in the event field (4) which essentially follows a constant event density.

2. The method according to claim 1, wherein the constant event density lies between 500 and 2000 events per 100 km length of product to be tested and preferably at 1000 events per 100 km length of product to be tested.

3. The method according to one of the preceding claims, wherein one or more further areas (5) connect to the area (4) representing the body of the product to be tested, and is or are likewise delimited by one or more further lines (51) in the event field (3), which in each case essentially follow a constant event density.

4. The method according to claim 3, wherein the event densities, which are followed by the line (41) delimiting the body (4) of the product to be tested and the further lines (51), essentially form a geometric sequence.

5. The method according to one of the preceding claims, wherein the area (4) representing the body of the product to be tested and/or the further areas (5) differ graphically from their respective surroundings, in particular by way of them having a different color, a different shade of gray and/or a different pattern than their respective surroundings.

6. The method according to one of the preceding claims, wherein the values of the parameter together with their extensions (L) in the longitudinal direction are represented as events (71, 72) in the event field (3).

7. The method according to claim 6, wherein no events are represented in the body (4) of the product to be tested or at least in a part of the body (4) of the product to be tested, said part being adjacent the abscissa (31) or to the straight line running parallel thereto.

8. The method according to claim 7, wherein the event field (3) is divided by a classification limit into two classification regions (81, 82), and no events are represented in a first classification region (81), and events (71, 72) are represented in a second classification region (82).

9. The method according to claim 8, wherein the classification limit essentially follows a constant event density.

10. A method for clearing defects (72) from an elongate textile product to be tested (9) which is moved along its longitudinal direction, wherein
readings of a characteristic of the textile product to be tested (9) along the longitudinal direction of the textile product to be tested (9) are detected,
values of a parameter of the textile product to be tested are determined from the readings,
an event field (3) is provided
which contains a quadrant or a part of a quadrant of a two-dimensional Cartesian coordinate system, whose abscissa (31) defines an extension (L) of parameter values in the longitudinal direction and whose ordinate (32) defines a deviation (ΔM) of the parameter from a desired value, and
which is divided by at least one clearing curve (6) into clearing regions (61, 62), of which a first clearing region (61) defines allowable events (71) and a second clearing region (62) defines unallowable events (72), and
densities of events in the event field (3) are determined from the values of the parameter and their extensions (L) in the longitudinal direction,
**characterised in that**
the textile product to be tested (9) is characterised according to one of the preceding claims and the characterization influences the setting of the clearing curve (6).

11. The method according to claim 10, wherein the first clearing region (61) and the second clearing region (62) graphically differ for one another, in particular by way of them having different colors, different shades of grey and/or different patterns.

12. The method according to claim 10 or 11, wherein the number of unallowable events (72) and/or the number of unallowable events (72) per length unit of the textile product to be tested (9) are computed on account of the readings of the yam parameter and on account of the clearing curve (6), and outputted.

13. The method according to one of the claims 10-12, wherein at least one first exclusion region (63) is defined in the first clearing region (61) and defines unallowable events, and/or at least one second exclusion region (64.1, 64.2) is defined in the second clearing region (62) and defines allowable events.

14. The method according to one of the preceding claims, wherein the event field (3) additionally contains at least one further quadrant or a part of a further quadrant.

15. The method according to one of the preceding claims, wherein the body (4) of the product to be tested, the further areas (5), the classification regions (81, 82) and/or the clearing regions (61, 62) are automatically adapted to the continuous detection of the readings and to the changes of densities of events (71, 72) in the event field (3), said changes resulting therefrom.

16. A device (1) for characterizing an elongate textile product to be tested (9) moved along its longitudinal direction, containing
a measurement head (11) for detecting readings of a characteristic of the textile product to be tested (9) along the longitudinal direction of the textile product to be tested (9) as well as for determining values of a parameter of the textile product to be tested from the readings, and
a control unit (14) connected to the measurement head (11), said control unit having
a memory unit and an output unit (15) for storing and outputting, respectively, of an event field (3), which contains a quadrant or a part of a quadrant of a two-dimensional Cartesian coordinate system, whose abscissa (31) defines an extension (L) of parameter values in the longitudinal direction and whose ordinate (32) defines a deviation (ΔM) of the parameter from a desired value, and
a computation unit for determining densities of events (71, 72) in the event field (3) from the values of the parameter and their extensions (L) in the longitudinal direction,
**characterised in that**
the control unit (14) is set up for outputting the body of the product to be tested as an area (4) in the event field (3) which is delimited
on the one hand by the abscissa (31) or a straight line running parallel thereto,
on the other hand by the ordinate (32) or a straight line running parallel thereto and further by a line (41) in the event field (3) which essentially follows a constant event density.

## Revendications

1. Procédé pour la caractérisation d'une matière textile allongée à contrôler (9) déplacée dans le sens de sa longueur, dans lequel
des valeurs de mesure d'une propriété de la matière textile à contrôler (9) sont acquises dans le sens longitudinal de la matière textile à contrôler (9),
des valeurs d'un paramètre de la matière textile à contrôler sont déterminées à partir des valeurs de mesure,
un champ d'événements (3) est produit, qui contient un quadrant ou une partie de quadrant d'un système de coordonnées cartésiennes à deux dimensions dont l'abscisse (31) indique une étendue (L) de valeurs de paramètre dans le sens longitudinal et l'ordonnée (32) un écart (ΔM) du paramètre par rapport à une valeur de consigne, et
des densités d'événements (71, 72) dans le champ d'événements (3) sont déterminées à partir des valeurs du paramètre et de son étendue (L) dans le sens longitudinal, **caractérisé en ce que**
dans le champ d'événements (3), un corps de matière à contrôler est représenté comme une aire (4) qui est délimitée
d'une part par l'abscisse (31) ou une droite parallèle à celle-ci,
d'autre part par l'ordonnée (32) ou une droite parallèle à celle-ci, et
en outre par une ligne (41) dans le champ d'événements (4) qui suit pour l'essentiel une densité d'événements constante.

2. Procédé selon la revendication 1, dans lequel la densité d'événements constante se situe entre 500 et 2000 événements par 100 km de longueur de matière à contrôler et de préférence à 1000 événements par 100 km de longueur de matière à contrôler.

3. Procédé selon l'une des revendications précédentes, dans lequel se raccordent à l'aire (4) représentant le corps de matière à contrôler une ou plusieurs autres aires (5) qui sont également délimitées par une ou plusieurs autres lignes (51) dans le champ d'événements (3) dont chacune suit pour l'essentiel une densité d'événements constante.

4. Procédé selon la revendication 3, dans lequel les densités d'événements suivies par la ligne (41) délimitant le corps de matière à contrôler (4) et les autres lignes (51) forment pour l'essentiel une suite géométrique.

5. Procédé selon l'une des revendications précédentes, dans lequel l'aire (4) représentant le corps de matière à contrôler et/ou les autres aires (5) se distinguent graphiquement de leur environnement, en particulier en ayant une autre couleur, un autre niveau de gris et/ou un autre motif que leur environnement.

6. Procédé selon l'une des revendications précédentes, dans lequel les valeurs du paramètre et leur étendue (L) dans le sens longitudinal sont représentées comme des événements (71, 72) dans le champ d'événements (3).

7. Procédé selon la revendication 6, dans lequel aucun événement n'est représenté dans le corps de matière à contrôler (4) ou au moins dans une partie du corps de matière à contrôler (4) contiguë à l'abscisse (31) ou à la droite parallèle à celle-ci.

8. Procédé selon la revendication 7, dans lequel le champ d'événements (3) est partagé par une limite de classification en deux zones de classification (81, 82) et aucun événement n'est représenté dans une première zone de classification (81) et des événements (71, 72) sont représentés dans une deuxième zone de classification (82).

9. Procédé selon la revendication 8, dans lequel la limite de classification suit sensiblement une densité d'événements constante.

10. Procédé pour le nettoyage de défauts (72) dans une matière textile allongée à contrôler (9) déplacée dans le sens de sa longueur, dans lequel
des valeurs de mesure d'une propriété de la matière textile à contrôler (9) sont acquises dans le sens longitudinal de la matière textile à contrôler (9),
des valeurs d'un paramètre de la matière textile à contrôler sont déterminées à partir des valeurs de mesure,
un champ d'événements (3) est produit
qui contient un quadrant ou une partie de quadrant d'un système de coordonnées cartésiennes à deux dimensions dont l'abscisse (31) indique une étendue (L) de valeurs de paramètre dans le sens longitudinal et l'ordonnée (32) un écart (ΔM) du paramètre par rapport à une valeur de consigne, et
qui est divisé par au moins une courbe de nettoyage (6) en zones de nettoyage (61, 62) parmi lesquelles une première zone de nettoyage (61) spécifie des événements admissibles (71) et une deuxième zone de nettoyage (62) des événements inadmissibles (72), et
des champ d'événements (3) sont déterminées à partir des valeurs du paramètre et de leur étendue (L) dans le sens longitudinal,
**caractérisé en ce que**
la matière textile à contrôler (9) est caractérisée selon l'une des revendications précédentes et
la caractérisation influe sur la définition de la courbe de nettoyage (6).

11. Procédé selon la revendication 10, dans lequel la première zone de nettoyage (61) et la deuxième zone de nettoyage (62) se distinguent graphiquement l'une de l'autre, en particulier en ayant des couleurs différentes, des niveaux de gris différents et/ou des motifs différents.

12. Procédé selon la revendication 10 ou 11, dans lequel le nombre d'événements inadmissibles (72) et/ou le nombre d'événements inadmissibles (72) par unité de longueur de la matière textile à contrôler (9) sont calculés sur la base des valeurs de mesure du paramètre du filé et sur la base de la courbe de nettoyage (6) et émis en sortie.

13. Procédé selon l'une des revendications 10 à 12, dans lequel est définie dans la première zone de nettoyage (61) au moins une première zone d'exclusion (63) qui spécifie des événements inadmissibles et/ou est définie dans la deuxième zone de nettoyage (62) au moins une deuxième zone d'exclusion (64.1, 64.2) qui spécifie des événements admissibles.

14. Procédé selon l'une des revendications précédentes, dans lequel le champ d'événements (3) contient en outre au moins un autre quadrant ou une partie d'un autre quadrant.

15. Procédé selon l'une des revendications précédentes, dans lequel le corps de matière à contrôler (4), les autres aires (5), les zones de classification (81, 82) et/ou les zones de nettoyage (61, 62) sont adaptées automatiquement à l'acquisition en cours de valeurs de mesure et aux modifications qui en résultent dans les densités des événements (71, 72) dans le champ d'événements (3).

16. Dispositif (1) pour la caractérisation d'une matière textile allongée à contrôler (9) déplacée dans le sens de sa longueur, comprenant
une tête de mesure (11) pour acquérir des valeurs de mesure d'une propriété de la matière textile à contrôler (9) dans le sens longitudinal de la matière textile à contrôler (9) et pour déterminer des valeurs d'un paramètre de la matière textile à contrôler à partir des valeurs de mesure, et
une unité de commande (14) reliée à la tête de mesure (11)
avec une unité de mémoire et une unité de sortie (15) pour la mémorisation et l'émission en sortie d'un champ d'événements (3) comprenant un quadrant ou une partie d'un quadrant d'un système de coordonnées cartésiennes à deux dimensions, dont l'abscisse (31) indique une étendue (L) de valeurs de paramètre dans le sens longitudinal et l'ordonnée (32) un écart (ΔM) du paramètre par rapport à une valeur de consigne, et
une unité de calcul pour déterminer des densités d'événements (71, 72) dans le champ d'événements (3) à partir des valeurs du paramètre et de leur étendue (L) dans le sens longitudinal,
**caractérisé en ce que**
l'unité de commande (14) est équipée pour émettre en sortie un corps de matière à contrôler sous la forme d'une aire (4) dans le champ d'événements (3) qui est délimitée
d'une part par l'abscisse (31) ou une droite parallèle à celle-ci,
d'autre part par l'ordonnée (32) ou une droite parallèle à celle-ci, et
en outre par une ligne (41) dans le champ d'événements (3) qui suit pour l'essentiel une densité d'événements constante.
